# EUROPEAN PATENT APPLICATION

(11) **EP 3 764 099 A1**
(43) Date of publication of application: **13.01.2021**
(21) Application number: 18908854.5
(22) Date of filing: 29.09.2018
(51) Int. Cl.: G01N 33/574, G01N 33/68

(54) **APPLICATION OF IGG4 DETECTION REAGENT IN PREPARATION OF COLORECTAL CANCER DIAGNOSTIC AGENT**

(30) Priority: 08.03.2018 CN 201810189540
(71) Applicant: Sun Yat-Sen University, Guangzhou, Guangdong 510275 (CN); The Sixth Affiliated Hospital of Sun Yat-Sen University, Guangzhou, Guangdong 510655 (CN)
(72) Inventor: LIU, Ruixian, Guangzhou, Guangdong 510655 (CN); YANG, Xiangling, Guangzhou, Guangdong 510655 (CN); CHEN, Junxiong, Guangzhou, Guangdong 510655 (CN); WEN, Chuangyu, Guangzhou, Guangdong 510655 (CN); WU, Feng, Guangzhou, Guangdong 510655 (CN); CHEN, Yongkang, Guangzhou, Guangdong 510655 (CN); WANG, Huihui, Guangzhou, Guangdong 510655 (CN); HUANG, Lanlan, Guangzhou, Guangdong 510655 (CN); LIU, Huanliang, Guangzhou, Guangdong 510655 (CN)
(74) Representative: J A Kemp LLP
(86) International application number: PCT/CN2018/108764
(87) International publication number: WO 2019/169857

(57) **Abstract**

The invention belongs to the field of biological medicine, and relates to use of an IgG4 detection reagent, a subclass of immunoglobulin, in preparation of a colorectal cancer diagnosis or prognosis reagent/kit. According to the present invention, IgG4, a subclass of immunoglobulin, is highly expressed in colorectal cancer with great difference and credibility. Use of IgG4 as a colorectal cancer diagnostic biomarker has advantages of being strong in specificity and high in sensitivity, which is beneficial to improving the diagnosis and treatment level of colorectal cancer, and effectively prevents and treats colorectal cancer.

## Description

### TECHNICAL FIELD

The invention belongs to the field of biomedicine, and relates to application of an IgG4 detection reagent in preparation of a colorectal cancer diagnosis reagent/kit and a colorectal cancer diagnosis reagent/kit.

### BACKGROUND

Colorectal cancer (CRC) is the most common malignant tumor of digestive tract and the second largest cancer in the world, with the third and fourth highest morbidity and mortality respectively. In recent years, with the improvement of people's living standards, the change of life style and the aging of the population, the incidence of colorectal cancer in China is gradually rising, ranking third in the incidence of malignant tumors and fifth in mortality in China, which is a serious threat to people's health and lives. Early detection, early diagnosis and early treatment are the key to the treatment of colorectal cancer. However, in the initial stage of colorectal cancer, most patients have no obvious symptoms, and go to see doctor with "enteritis" as the disease progressing, so that most patients with colorectal cancer are already in the stage of tumor progression at the time of diagnosis, lose the best time for treatment, resulting in a poor prognosis with five-year survival rate less than 20%. Therefore, to explore the early, rapid and large-scale diagnosis of colorectal cancer and improve the level of diagnosis and treatment is an urgent problem to prevent and treat colorectal cancer.

At present, the widely used clinical diagnosis methods of colorectal cancer are fecal occult blood test (FOBT), flexible sigmoidoscopy, colonoscopy and CT colonography. However, these detection methods have certain limitations. For example, the process of fecal examination and separation is tedious and easy to be disturbed by bacteria, food and intestinal mucus. At the same time, its specificity is relatively low and the false positive rate is relatively high. In addition, its usefulness as a screening tool is limited because of the unpleasant nature; colonoscopy is the current gold standard and has high specificity, but because of its invasiveness and certain risk of complications, and high technical requirements for testing equipment and testing personnel, the compliance of colonoscopy is not high, and the misdiagnosis rate is high. Blood is more accessible than fecal, tissue or colonoscope invasion, is more acceptable to patients, which is of greater value as a vehicle for early screening. Because of fast, convenient and safe sampling, the detection of serum tumor markers has become a common method for early tumor diagnosis, identification, staging guidance and tumor recurrence and metastasis.

Human serum contains a large amount of immunoglobulin (Ig). Immunoglobulin is a large Y-shaped protein secreted mainly by plasma cells and used by the immune system to identify and neutralize foreign substances such as bacteria, viruses and other pathogens, with a variety of immunoglobulin subclasses; more importantly, different subtypes of immunoglobulin fluctuate when the body is infected; compared with other protein molecules, immunoglobulin has more stable properties and longer half-life, which makes the expression levels of different immunoglobulin subclasses in plasma have broad prospects in the diagnosis of diseases such as infection and tumor.

IgG4 is a subclass of IgG, the concentration of IgG4 is increased in autoimmune pancreatitis, and is currently used as one of the markers of autoimmune pancreatitis (AIP). Some scholars have also found that there is a certain relationship between IgG4 and inflammatory bowel disease (IBD). The pathogenesis of the disease is ever-changing, and the occurrence of different organs, tissues and different types of diseases are different, often the role of the same marker in different tissues is different or even the opposite. Without evidence, the role of different molecules in different tissues cannot be simply inferred.

### SUMMARY OF THE DISCLOSURE

The invention aims to provide a molecular marker of colorectal cancer with strong specificity and high sensitivity, a diagnostic reagent using the molecular marker and application thereof.

The above-mentioned object of the present invention is achieved by the following technical means:
In one aspect, the invention provides the application of IgG4 detection reagent in preparation of colorectal cancer diagnosis reagent/kit.

According to the invention, blood samples of healthy people (preferably plasma samples) and plasma samples of colorectal cancer patients are collected for detection, standardized abundance of different plasma pro/antiinflammatory factors is obtained through data processing analysis, and it is not obvious that immunoglobulin subclass-IgG4 is differentially expressed and can be used as a biomarker for colorectal cancer diagnosis.

The IgG4 detection reagent is used for detecting the expression amount of the mRNA of the IgG4; or detecting the expression amount of the IgG4 protein, or detecting one or more of the biological activities of the IgG4 protein. As a preferred embodiment of the present invention, the IgG4 detection reagent detects the expression amount of the IgG4 protein.

As a preferred embodiment, the expression amount of IgG4 represents the concentration of IgG4 in the detection sample; as a more preferred embodiment, the concentration of IgG4 in plasma is indicated.

As a preferred embodiment, the detection reagent is an antibody, an antibody functional fragment, or a conjugated antibody.

Among them, the antibody may be a monoclonal antibody, a polyclonal antibody, a multivalent antibody, a multi-specific antibody (e.g. bispecific antibody), and/or antibody fragments linked to the proteasome. The antibody can be a chimeric antibody, a humanized antibody, a CDR-grafted antibody, or a human antibody. Antibody fragments can be, for example, Fab, Fab', F(ab') 2, Fv, Fd, single chain Fv (scFv), disulfide-bonded Fv (sdFv), or VL, VH domains. The antibody may be in a conjugated form, for example, bound to a label, a detectable label, or a cytotoxic agent.

As a preferred embodiment, the kit is an ELISA kit, further, an ELISA kit based on an anti-human IgG4-conjugated antibody for detecting the expression amount of the IgG4 protein.

The detection method of the detection reagent is any one or more of ELISA method, protein chip method, liquid chromatography, immunoturbidimetry and flow cytometry; in a preferred embodiment, the detection method of the detection reagent is one or more of a protein chip method, an ELISA method or an immunoturbidimetry method.

The risk of colorectal cancer is high when the expression amount of the IgG4 protein ≥ 604.1 µg/ml, and the risk of colorectal cancer is low when the expression amount of the IgG4 protein ≤ 591.4 µg/ml is detected. The risk is uncertain when the expression amount of the IgG4 protein is between the two, and it is recommended to supplement other existing or newly developed diagnostic methods for disease screening.

As an exemplary embodiment of the present invention, the calculation method of the expression amount of IgG4 may employ a standard curve method.

As a preferred embodiment, the sample detected by the detection reagent is blood; as a preferred embodiment, the test sample is plasma.

In another aspect, the present invention also provides a reagent/kit for the diagnosis of rectal cancer, the reagent/kit comprising an IgG4 detection reagent. The IgG4 detection reagent is as described above.

In another aspect, the present invention also provides a chip for colorectal cancer diagnosis, the chip comprises a solid phase carrier and a probe of a biomarker IgG4 fixed on the solid phase carrier. As a preferred embodiment, the chip is a protein chip.

In another aspect, the present invention also provides a diagnosis system for colorectal cancer, the detection system comprises:
a) a detection component: the detection component is used for detecting the expression amount of IgG4 of the diagnostic subject; and
b) a result judgment component: the result judgment component is used for obtaining the possibility or risk value of suffering from the colorectal cancer according to the expression amount of IgG4 detected by the detection component.

As a preferred embodiment, the detection component is one or more of a microplate reader, a laser scanner, a flow cytometer and a liquid chromatograph; as a preferred embodiment, the detection component is one or more of a microplate reader and a laser scanner.

As a preferred embodiment, the result judgment component is software which comprises an input module, an analysis module and an output module; the input module is used for inputting the expression amount of IgG4; the analysis module is used for analyzing the possibility or risk value of suffering from colorectal cancer according to the expression amount of IgG4; the output module is used for outputting the analysis result of the analysis module.
wherein, in the system, the expression amount of IgG4 is the expression amount of the mRNA of the IgG4; or the expression amount of the IgG4 protein. As a preferred embodiment, the detection component detects the expression amount of the IgG4 protein.

As a preferred embodiment, in the structure judgment component, the risk of colorectal cancer is high when the expression amount of the IgG4 protein ≥ 604.1 µg/ml, and the risk of colorectal cancer is low when the expression amount of the IgG4 protein ≤ 591.4 µg/ml is detected.

Wherein the diagnostic sample of the diagnostic system is blood; more preferred, plasma.

The invention has the beneficial effects that:
(1) The invention finds that among a large number of immunoglobulin subclasses, IgG4 is differentially expressed, which can be used as a biomarker in colon and rectum, and has good specificity and sensitivity.
(2) The detection object of the detection reagent of the invention is blood. Compared with the existing fecal occult blood test (FOBT), the process of fecal examination and separation is tedious and easy to be disturbed by bacteria, food and intestinal mucus; at the same time, its specificity is relatively low and the false positive rate is relatively high and it has the unpleasant nature. Blood is more accessible, and is more acceptable to patients, which is of greater value as a vehicle for early screening.
(3) Compared with other proteins in plasma, the immunoglobulin has longer half-life and stable properties, and is more suitable for being used as a marker for disease diagnosis.

### BRIEF DESCRIPTION OF THE DRAWINGS

FIG. 1 is a volcano plot of differential immunoglobulin subclasses.
FIG. 2 is a comparison of IgG4 concentration in plasma of 25 healthy subjects and 35 colorectal cancer patients.
FIG. 3 is a gradient dilution plot of an immunoglobulin standard.

### DETAILED DESCRIPTION

The technical solution of the present invention is further illustrated by the following specific examples, which are not intended to limit the scope of the present invention. Other non-essential modifications and adaptations of the invention by others according to the inventive concept remain within the scope of the invention.

### Example 1 Expression analysis of differential immunoglobulin subclasses

### Experimental method:

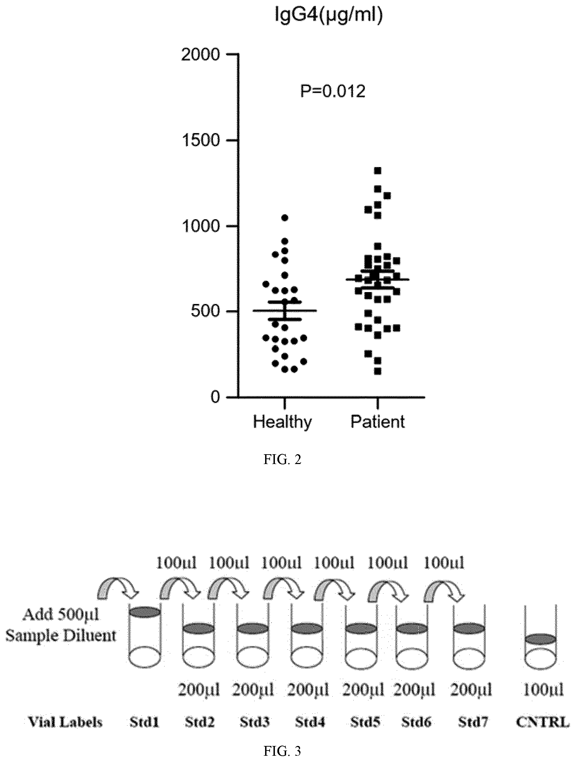

The invention first collects the plasma of 25 healthy people and 35 colorectal cancer patients in the Sixth Affiliated Hospital, Sun Yat-sen University, and selects the differential immunoglobulin subclasses through the following steps:
1. Collecting 3-5 cm of whole blood of a healthy person or a colorectal cancer patient through a sodium citrate anticoagulant blood collection tube;
2. Centrifuging for 10 mins at rotational speed of 500 g, and the upper plasma layer is obtained by sucking up after the blood cells are precipitated;
3. Collecting the plasma supernatant, centrifuging for 15 mins at rotational speed of 2000 g to obtain the plasma sample after precipitation being removed;
4. Subpackaging and freezing in a -80 °C refrigerator;
5. Detecting the plasma sample by a Raybiotech protein chip (QAH-ISO-1);
   (1) Complete drying of slide chips
      Taking out the slide chip from the box, balancing at room temperature for 20-30 mins, opening the bag, opening the seal, and placing the chip in a vacuum dryer or dry at room temperature for 1-2 h.
   (2) Formulation of standards
      Gradient dilutions of immunoglobulin subclass standards are shown in FIG. 3.
      Adding 500 µL of sample diluent to the tubules of the standard mixture of immunoglobulin subclasses and redissolving the standards. Before opening the tubule, centrifuging quickly, gently blowing up and down to the dissolve the powder, and marking the tubule as Std1.
      Labeling 6 clean centrifuge tubes as Std2, Std3 to Std7, and adding 200 µL sample diluent to each tubule.
      Pipetting 100 µL of Std1 into Std2 to mix gently, then pipetting 100 µL from Std2 for adding into Std3, and so on gradient diluting until Std7.
      Pipetting 100 µL of sample diluent into another new centrifuge tube and marked as CNTRL, as a negative control.
      Note: because the initial concentration of each immunoglobulin subclass is different, the series concentration of each cytokine is different after gradient dilution from Std1 to Std7.
   (3) Operation process of chip
      (a) Adding 100 µL of sample dilution to each well and incubating for 1 h at room temperature on a shaker to block the quantitative antibody chip.
      (b) Removing buffer from each well, adding 100 µL of standards and samples to each well, and incubating at 4 °C overnight. (the sample is diluted 40,000 times)
      (c) Washing slides by Thermo Scientific Wellwash Versa plate washer, which was divided into two steps. Firstly, washing the slides with 1 × wash solution I, and washing with 250 µL of 1 × wash solution I per well for 10 times, each time shaking for 10 s, the shaking intensity was chosen to be high, and diluting the wash solution 120 times (20 ×) with deionized water. Then, change to wash the slides with 1 × wash solution II channel, washing with 250 µL of 1 × wash solution II per well for 6 times, each time shaking for 10 s, the shaking intensity was chosen to be high, and diluting the wash solution II 20 times (20 ×) with deionized water.
      (d) Detecting the incubation of the antibody mixture, and detecting the antibody mixture tubules by centrifugation, then adding 1.4 mL of the sample diluent, mixing evenly, and centrifuging quickly again. Adding 80 µL of detection antibody to each well and incubating on RT shaker for 2 h.
      (e) Washing, same as step (c)
      (f) Incubating Cy3 -streptavidin, centrifuging the Cy3 -streptavidin tubules, followed by adding 1.4 ml of sample diluent, mixing well and centrifuging quickly again. Adding 80 µL of Cy3-streptavidin to each well, cover the slide with aluminum foil to incubate in the dark, and incubate on a RT shaker for 1 h.
      (g) Washing, same as step (c)
      (h) Fluorescence detection, using a laser scanner such as an InnoScan 300 scanning signal, using Cy3 or green channel (excitation frequency = 532 nm), instrument model: InnoScan 300 Microarray Scanner, manufacturer: Innopsys, scan parameters: WaveLengh: 532 nm; Resolution: 10 µm, data analysis was performed by using QAH-ISO-1 data analysis software.
6. The detection data were standardized, and the expression of each immunoglobulin subclass in two groups of samples was compared and analyzed.

### Analytical Data:

### 1. Data quality control

After plotting the standard curves for the different antibody types, we obtained the best confident range for each antibody subclass, and samples above the maximum (% above Max) or below the limit of detection (% below LOD) can affect confidence (% in best confidence). The following table represents the quality of different antibody subclass readings at one dilution (40,000).

**Table 1 Quality Control of Different Antibody Subclass Concentration Detection**

| **Target** | **LOD (pg/mL)** | **Best Confident Range** | **Maximum Value** | **Highest Reported Value** | **Lowest Reported Value** | **% *Below LOD*** | **% Above LOD but < 3*x* LOD** | **% in Best Confidence** | **% Above Maximum** |
|---|---|---|---|---|---|---|---|---|---|
| IgA | 83.9 | 251.6 - | 200,000 | 43,640.4 | 321.2 | *0.0* | *0.0* | **100.0** | *0.0* |
| IgD | 269.9 | 809.6 - 50,000 | 50,000 | 14,165.2 | 237.8 | *3.3* | *3.3* | **93.3** | *0.0* |
| IgE | 235.2 | 705.5 - 50,000 | 50,000 | 1,683.6 | 0.0 | **38.3** | **33.3** | **28.3** | *0.0* |
| IgM | 61.6 | 184.8 - | 100,000 | 66,289.3 | 11,008.8 | *0.0* | *0.0* | **100.0** | *0.0* |
| IgG1 | 516.0 | 1548.1 - | 400,000 | 583,034.6 | 7,250.7 | *0.0* | *0.0* | **93.3** | 6.7 |
| IgG2 | 928.7 | 2786.2 - | 400,000 | 163,712.1 | 1,712.5 | *0.0* | 8.3 | **91.7** | *0.0* |
| IgG3 | 31.1 | 93.2 - 50,000 | 50,000 | 8,888.1 | 801.6 | *0.0* | *0.0* | **100.0** | *0.0* |
| IgG4 | 18.3 | 55 - 50,000 | 50,000 | 33,074.9 | 3,858.4 | *0.0* | *0.0* | **100.0** | *0.0* |
| 0 | 0.0 | 0 - 0 | 0 | #N/A | #N/A | #N/A | #N/A | #N/A | #N/A |
| 0 | 0.0 | 0 - 0 | 0 | #N/A | #N/A | #N/A | #N/A | #N/A | #N/A |

As can be seen from Table 1: the level of IgE in whole blood plasma was lower, the concentration of about 38.3% of the sample was lower than the limit of detection, and the reliability was lower. The reliability of other antibody subclasses were high (greater than 90%).

### 2. Comparison of plasma IgG4 concentration between healthy people and patients with intestinal cancer

Plasma IgG concentrations were compared between 25 healthy people and 35 colorectal cancer patients (see FIG. 2).

Mean value of healthy people group = 503.6 µg/ml, SEM = 51.26, confidence interval (403.1-604.1)

Mean value of patients with intestinal cancer = 686.4 µg/ml, SEM = 48.48, confidence interval (591.4-781.4)

P is calculated by T-test.

### 3. Differential Immunoglobulin Volcano Plot Analysis

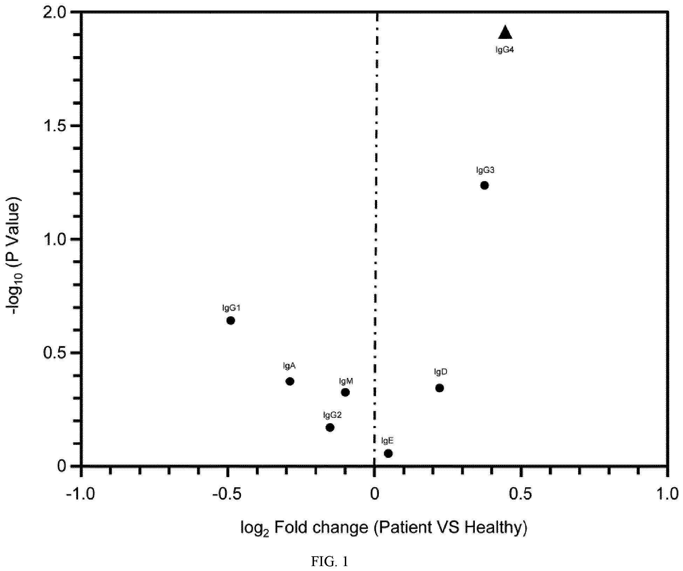

The results of differential immunoglobulin volcano plot analysis are shown in Table 2 and FIG. 1.

### Differentially expressed proteins (DEPs)

In order to detect differentially expressed proteins, we compared the concentration values of different antibody subclasses in the samples diluted by 40000 times, and obtained the log2 fold change (log2FC) of each antibody subclass and the P value after T test (-log10 P value, p value) between the two groups.

Differentially expressed protein refers to a protein having a P value less than 0.05 (-log10 P value greater than 1.3) and a fold change greater than 1.2 fold or less than 0.83 fold.

**Table 2 Differential immunoglobulin concentration values**

| | **Mean-E** | **Mean-C** | **log2FC** | **p value** |
|---|---|---|---|---|
| IgG4 | 17160.89 | 12590.72 | 0.4467327 | 0.012285 |

From Table 2 and Description: compared with the healthy people group (group C), mean value = 12590.72 pg/ml, there is a significantly high expression of IgG4 immunoglobulin subclass in colorectal cancer group (group E), mean value = 17160.89 pg/ml, p value was about 0.012.

As can be seen from FIG. 1, among the various immunoglobulin subclasses, the plasma concentration of the IgG4 (triangle) immunoglobulin subclass is significantly different in healthy people. The remaining immunoglobulin subclasses, in addition to IgE, have a high degree of confidence, but are not different between the healthy people group and the colorectal cancer patient group (see volcano plot in FIG. 1) and cannot be used as markers for the diagnosis of colorectal cancer.

### 4. Sensitivity and specificity

According to the confidence interval of IgG4 concentration in plasma of healthy people and patients with intestinal cancer in 2, it can be seen that the risk of colorectal cancer is high when the expression amount of the IgG4 protein ≥ 604.1 µg/ml, and the risk of colorectal cancer is low when the expression amount of the IgG4 protein ≤ 591.4 µg/ml is detected. We compared the IgG4 concentration of 25 healthy people and 35 colorectal cancer patients with this range, and obtained the sensitivity of IgG4: 23/35 = 65.7%, specificity: 15/25 = 60%.

Compared with the single serological factor detection in the prior art, , IgG4 has higher sensitivity and higher clinical practical significance than CEA and CA50, which are common in colorectal cancer.

The invention finds that IgG4 can be used as a marker in plasma, and a better pre-diagnosis effect can be achieved by the way of blood sampling, which is of great significance for reducing the sampling pain of the people to be detected and realizing more popular pre-diagnosis and screening.

## Claims

1. Use of an IgG4 detection reagent in preparation of colorectal cancer diagnosis reagent/kit.

2. The use of claim 1, **characterized in that** the IgG4 detection reagent is for detecting the expression amount of mRNA of IgG4; detecting the expression amount of the IgG4 protein or detecting the biological activity of the IgG4 protein; preferably, the detecting of the expression amount of the IgG4 protein.

3. The use of claim 1, **characterized in that** the detection reagent is an antibody, an antibody functional fragment or a conjugated antibody for detecting IgG4; preferably, the conjugated antibody is a fluorescein conjugated antibody or a bio-enzyme conjugated antibody.

4. The use of claim 1, **characterized in that** the kit is an ELISA kit.

5. The use of claim 1, **characterized in that** the detection method of the detection reagent is any one or more of ELISA method, protein chip method, liquid chromatography, immunoturbidimetry and flow cytometry; preferably, a protein chip method or an ELISA method.

6. The use of claim 1, **characterized in that**, the risk of colorectal cancer is high if the detection result of the detection reagent is that the expression amount of the IgG4 protein ≥ 604.1 µg/ml; the risk of colorectal cancer is low if the detection result of the detection reagent is that the expression amount of the IgG4 protein ≤ 591.4 µg/ml.

7. The use of claim 1, **characterized in that** the detection sample of the detection reagent is blood; preferably, plasma.

8. A reagent/kit for colorectal cancer diagnosis, the reagent/kit comprises an IgG4 detection reagent; preferably, the reagent/kit comprises an antibody, an antibody functional fragment or a conjugated antibody for detecting IgG4; more preferably, the conjugated antibody is a fluorescein conjugated antibody or a bio-enzyme conjugated antibody.

9. A chip for colorectal cancer diagnosis, **characterized in that** the chip comprises a solid phase carrier and a probe of a biomarker IgG4 fixed on the solid phase carrier; preferably, the chip is a protein chip.

10. A diagnosis system for colorectal cancer, **characterized in that** the detection system comprises:
a) a detection component: the detection component is used for detecting the expression amount of IgG4 of the diagnostic subject;
b) a result judgment component: the result judgment component is used for obtaining the possibility or risk value of suffering from the colorectal cancer according to the expression amount of IgG4 detected by the detection component;
preferably, the detection component is one or more of a microplate reader, a laser scanner, a flow cytometer and a liquid chromatograph; more preferably, the detection component is one or two of a laser scanner and a microplate reader;
preferably, the result judgment component is software which comprises an input module, an analysis module and an output module; the input module is used for inputting the expression amount of IgG4; the analysis module is used for analyzing the possibility or risk value of suffering from colorectal cancer according to the expression amount of IgG4; the output module is used for outputting the analysis result of the analysis module;
preferably, the expression amount of IgG4 is the expression amount of the mRNA of IgG4; or the expression amount of the IgG4 protein; more preferably, the expression amount of the IgG4 protein;
preferably, the diagnostic sample of the diagnostic system is a blood sample; more preferably, a plasma sample; preferably, in the structure judgment component, the risk of colorectal cancer is high when the expression amount of the IgG4 protein ≥ 604.1 µg/ml, and the risk of colorectal cancer is low when the expression amount of the IgG4 protein ≤ 591.4 µg/ml.
